# EUROPÄISCHE PATENTANMELDUNG

(11) **EP 4 406 725 A2**
(43) Veröffentlichungstag der Anmeldung: **31.07.2024**
(21) Anmeldenummer: 23214612.6
(22) Anmeldetag: 06.12.2023
(51) Int. Cl.: B29C 49/42, F16K 31/122, A61L 2/20, B29C 49/12, B29C 49/36, B29C 49/46, B29C 49/78, B29L 31/00, F16K 31/126

(54) **VORRICHTUNG ZUM UMFORMEN VON KUNSTSTOFFVORFORMLINGEN ZU KUNSTSTOFFBEHÄLTNISSEN MIT STERILISIERBAREM ASEPTIKVENTIL**

(30) Priorität: 25.01.2023 DE 102023101834
(71) Anmelder: Krones AG, 93073 Neutraubling (DE)
(72) Erfinder: Habenschaden, Nina, 93073 Neutraubling (DE); Buess, Armin, 93073 Neutraubling (DE); Senn, Konrad, 93073 Neutraubling (DE); Hoellriegl, Thomas, 93073 Neutraubling (DE)
(74) Vertreter: Bittner, Bernhard

(57) **Zusammenfassung**

Eine Vorrichtung zum Umformen von Kunststoffvorformlingen (10) zu Kunststoffbehältnissen (15), mit einer Transporteinrichtung (2), welche die Kunststoffvorformlinge (10) entlang eines vorgegeben Transportpfads transportiert, **wobei** die Transporteinrichtung (2) einen Transportträger (22) aufweist, an dem eine Vielzahl von Umformungsstationen (20, 4) zum Umformen der Kunststoffvorformlinge (10) aufweist, **wobei** diese Umformungsstationen (20, 4) jeweils Beaufschlagungseinrichtungen aufweisen, welche die Kunststoffvorformlinge (10) zu ihrer Expansion mit einem fließfähigen und gasförmigen Medium beaufschlagen und **wobei** die Vorrichtung wenigstens ein erstes Druckluftreservoir aufweist, welches dazu geeignet und bestimmt ist, Druckluft unter einem ersten vorgegebenen Druck (P2) zu speichern und jede Umformungsstation (20) eine Ventilanordnung mit einer Vielzahl von Ventileinrichtungen aufweist, um die Kunststoffvorformlinge (10) mit wenigstens zwei Drücken zu beaufschlagen, **wobei** wenigstens eine dieser Ventileinrichtungen eine pneumatisch gesteuerte Ventileinrichtung (50) ist, **dadurch gekennzeichnet**, dass diese Ventileinrichtung (50) einen Arbeitsraum (56) zur Aufnahme des zur Beaufschlagung der Kunststoffvorformlinge (10) verwendeten gasförmigen Mediums aufweist sowie einen zwischen wenigstens zwei Position bewegbaren Ventilkolben (54) und die Ventileinrichtung (50) weiterhin einen Steuerraum (58) aufweist, der von einem gasförmigen Steuermedium zur Bewegung des Ventilkolbens beaufschlagbar ist und **wobei** zwischen dem Arbeitsraum (56) und dem Steuerraum (58) ein Übergangsbereich (60) angeordnet.

## Beschreibung

Die vorliegende Erfindung bezieht sich auf eine Vorrichtung und ein Verfahren zum Umformen von Kunststoffvorformlingen zu Kunststoffbehältnissen. Derartige Vorrichtungen und Verfahren sind aus dem Stand der Technik seit langem bekannt. Dabei werden üblicherweise erwärmte Kunststoffvorformlinge durch Beaufschlagung mit Druckluft zu Kunststoffbehältnissen und insbesondere Kunststoffflaschen umgeformt. Zu diesem Zweck werden die Kunststoffvorformlinge mit wenigstens zwei, neuerdings mit mehreren unterschiedlichen Druckniveaus beaufschlagt. Üblicherweise ist dabei eine Vielzahl von Umformungsstationen an einen drehbaren Träger, dem sogenannten Blasrad angeordnet. Die einzelnen Umformungsstationen weisen dabei jeweils Ventilblöcke bzw. Ventilanordnungen auf, die eine Vielzahl von Ventilen aufweisen. Bei diesen Ventilen handelt es sich teilweise um pneumatisch gesteuerte Ventile.

In jüngerer Zeit sind auch Blasformmaschinen oder Umformungseinrichtungen bekannt geworden, welche die Behältnisse unter aseptischen oder sterilen Bedingungen umformen. Dies bedeutet, dass der Umformungsvorgang auch mit Sterilluft durchgeführt wird.

Dies bedeutet, dass im Stand der Technik aber bevorzugt auch im Falle der Erfindung die Blasformmaschine in einem von einem Produktionsbetrieb unterschiedlichen Sterilisationsbetrieb mit einem Sterilisationsmedium, wie zum Beispiel Wasserstoffperoxid, sterilisiert wird. Nach dem Sterilisationsbetrieb werden die Behältnisse unter sterilen Bedingungen mit Sterilluft, insbesondere mit gefilterter Luft, umgeformt. Während dem Sterilisationsbetrieb werden auch die Ventilblöcke bzw. die Ventile mit Sterilisationsmedium sterilisiert.

Während dem Arbeitsbetrieb, ist die jeweilige Steuerluft zum Ansteuern der Ventile jedoch üblicherweise nicht steril.

Bei Ventilanordnungen oder Ventilblöcken in einem Sterilraum oder allgemein einem aseptischen Raum sollte gewährleistet sein, dass die zu schaltende aseptische Blasluft nicht mit der Steuerluft der Ventile verunreinigt wird. Aus diesem Grund wird im Stand der Technik teilweise eine hermetische Abdichtung der von der Blasluft durchströmten Bereiche gegenüber der Steuerluft gefordert. Zu diesem Zweck kann beispielsweise das Hubventil mittels eines Faltenbalgs oder einer Membran hermetisch zum Ventilgehäuse abgedichtet werden. Aus dem Stand der Technik bekannte Blasblöcke oder Ventilanordnungen sind nicht sterilisierbar, da zum einen die Sterilgrenzen nicht definiert sind und zum anderen die Materialien nicht passend ausgelegt sind. Insbesondere sind im Stand der Technik Ventileinrichtung nicht sterilisierbar, wenn sie keine trennende Membran oder einen trennenden Balg aufweisen.

In der Praxis kann es vorkommen, dass derartige Faltenbälge reißen. Daneben haben derartige Ventile den Nachteil, dass sie einen größeren Bauraum im Vergleich zu einem balglosen Ventil benötigen. Daneben sind üblicherweise die Geometrien derartiger Bälge oder die Werkstoffe an ihren Belastungsgrenzen. Weiterhin wird das Material des Balges nach der Sterilisation durch eine hohe Temperatur weich, sodass bei einem sofortigen Produktionsstart hierdurch Defekte an dem Balg entstehen.

Daneben benötigt ein derartiges Ventil zwei Piloten und/oder Steuerdrücke zum Öffnen und Schließen, was zu höheren Kosten und einem höheren Platzbedarf führt.

Der vorliegenden Erfindung liegt daher die Aufgabe zugrunde, eine Blasformmaschine zur Verfügung zu stellen, welche insbesondere für Sterilraumanwendungen vielseitiger einsetzbar ist, welche jedoch weiterhin auch den eigentlichen Blasvorgang, insbesondere bei der Beaufschlagung mit unterschiedlichen Drücken vereinfacht. Dies wird erfindungsgemäß durch die Gegenstände der unabhängigen Patentansprüche erreicht. Vorteilhafte Ausführungsformen und Weiterbildungen sind Gegenstand der Unteransprüche.

Eine erfindungsgemäße Vorrichtung zum Umformen von Kunststoffvorformlingen zu Kunststoffbehältnissen weist eine Transporteinrichtung auf, welche die Kunststoffvorformlinge entlang eines vorgegebenen Transportpfads transportiert. Dabei weist die Transporteinrichtung einen (bewegbaren und insbesondere drehbaren) Transportträger auf, an dem eine Vielzahl von Umformungsstationen zum Umformen der Vorformlinge zu Kunststoffbehältnissen angeordnet ist, wobei diese Umformungsstationen jeweils Beaufschlagungseinrichtungen aufweisen, welche die Kunststoffvorformlinge zu ihrer Expansion mit einem fließfähigen und insbesondere gasförmigen Medium beaufschlagen (und/oder hierzu geeignet und bestimmt sind).

Daneben weist die Vorrichtung wenigstens ein erstes Druckluftreservoir auf, welches dazu geeignet und bestimmt ist, Druckluft unter einem ersten vorgegebenen Druck zu speichern und jede Umformungsstation weist eine Ventilanordnung mit einer Vielzahl von Ventileinrichtungen auf, um die Kunststoffvorformlinge mit wenigstens zwei (insbesondere unterschiedlichen) Drücken zu beaufschlagen.

Weiterhin ist wenigstens eine dieser Ventileinrichtungen eine pneumatisch gesteuerte Ventileinrichtung.

Erfindungsgemäß weist diese Ventileinrichtung einen Arbeitsraum zur Aufnahme des zur Beaufschlagung der Kunststoffvorformlinge verwendeten gasförmigen Mediums auf sowie einen zwischen wenigstens zwei Positionen bewegbaren Ventilkolben. Weiterhin weist die Ventileinrichtung einen Steuerraum auf, der von einem gasförmigen Steuermedium zur Bewegung des Ventilkolbens beaufschlagbar ist. Dabei ist zwischen dem Arbeitsraum und dem Steuerraum wenigstens ein Übergangsbereich angeordnet.

Bevorzugt dient dieser Übergangsbereich zur Abgrenzung bzw. Trennung des Arbeitsraums und des Steuerraums. Auf diese Weise kann der Steuerraum mit unsteriler Luft beaufschlagt werden, ohne dass diese zu dem Arbeitsraum gelangt.

Besonders bevorzugt weist dieser Übergangsbereich einen Übergangsraum auf. Dieser Übergangsraum kann dabei beispielsweise im Betrieb auch mit einem sterilen Medium befüllt werden. Der Übergangsbereich wirkt daher in der Art eines Grauraums, der den aseptischen Arbeitsraum von dem nicht-aseptischen Steuerraum trennt. Bevorzugt kann damit der Steuerraum auch mit nichtsteriler Luft beaufschlagt werden. Der Übergangsbereich dient bevorzugt als Hygienebrücke.

Damit wird eine Ventileinrichtung vorgeschlagen, welche aseptisch und bevorzugt balglos ausgebildet ist und welche bevorzugt durch mindestens drei Zonen definiert bzw. aufgebaut ist. Bevorzugt befindet sich in einer dieser Zonen der Arbeitsraum, in einer weiteren der Steuerraum und bevorzugt ist zwischen diesen zwei Zonen und/oder Räumen eine Übergangszone angeordnet.

Damit handelt es sich bevorzugt bei der ersten Zone um einen Aseptikraum, in dem die aseptische Prozessluft strömen kann, wobei die Ventileinrichtung dazu geeignet und bestimmt ist, diesen Aseptikraum bzw. den Arbeitsraum mit dem zu expandierenden Behältnis in Verbindung zu bringen oder davon zu trennen.

Besonders bevorzugt sind der Arbeitsraum und/oder der Übergangsbereich und bevorzugt der Arbeitsraum und der Übergangsbereich sterilisierbar. Damit ist sowohl der Arbeitsraum bzw. diese Zone mit dem Arbeitsraum sterilisierbar. Auch die Übergangszone, welche, wie oben erwähnt einen Grauraum bildet, ist bevorzugt sterilisierbar.

Bei einer weiteren bevorzugten Ausführungsform ist der Steuerungsraum gegenüber dem Übergangsbereich mittels wenigstens einer ersten Dichtungseinrichtung abgedichtet. Bei dieser Dichtungseinrichtung kann es sich beispielsweise um einen Dichtring handeln.

Bei einer weiteren bevorzugten Ausführungsform ist der Übergangsbereich gegenüber dem Arbeitsraum mit wenigstens einer zweiten Dichtungseinrichtung abgedichtet. Auch bei dieser zweiten Dichtungseinrichtung handelt es sich bevorzugt um einen Dichtungsring.

Besonders bevorzugt sind die erste Dichtungseinrichtung und die zweite Dichtungseinrichtung voneinander in einer Längsrichtung der Ventileinrichtung und/oder einer Bewegungsrichtung der Kolbeneinrichtung.

Besonders bevorzugt ist zwischen der ersten Dichtungseinrichtung und der zweiten Dichtungseinrichtung wenigstens eine weitere Dichtungseinrichtung und bevorzugt zwei weitere Dichtungseinrichtungen angeordnet.

Bei einer weiteren vorteilhaften Ausführungsform ist eine erste Dichtungseinrichtung, welche den Steuerraum gegenüber dem Dichtungsbereich und/oder dem Übergangsbereich abdichtet, derart angeordnet, dass sie niemals einen aseptischen Bereich der Ventilanordnung kontaktiert (insbesondere auch unabhängig von einer Stellung des Ventilkolbens.

Bevorzugt ist dabei der genannte Grauraum bzw. der Übergangsbereich durch mindestens eine Dichtungseinrichtung von dem Aseptikraum bzw. dem Arbeitsraum getrennt. Wie erwähnt ist dabei bevorzugt dieser Grauraum sterilisierbar.

Weiterhin ist wie oben erwähnt, ein durch mindestens eine Dichtungseinrichtung von diesem Übergangsbereich abgegrenzter Steuerraum vorgesehen, welcher (insbesondere bei einem Steuerdruckwechsel) bevorzugt die Kolbeneinrichtung bewegt (beispielsweise von einer geschlossenen in eine geöffnete Stellung oder umgekehrt).

Wie erwähnt, sind dabei die Dichtungseinrichtungen dieses Steuerraums derart ausgeführt, dass sie niemals eine Oberfläche des Aseptikraums, d.h. des Arbeitsraums kontaktieren.

Dieser Übergangsbereich dient bevorzugt sowohl als Hygienebrücke als auch besonders bevorzugt als Druckfeder zum Vorspannen der Kolbeneinrichtung.

Bei einer weiteren vorteilhaften Ausführungsform ist eine weitere Ventileinrichtung und insbesondere ein Pilotventil vorgesehen. Dieses Pilotventil steuert bevorzugt die Steuerluft für das Öffnen der Kolbeneinrichtung. Weiterhin kann ein geringerer Pilotdruck dazu dienen, den Kolben beispielsweise über eine Luftfeder zu schließen und/oder in eine geschlossene Stellung der Ventileinrichtung zu bewegen.

Der Raum, der mit der Luftfeder gesteuert wird, kann bevorzugt zusätzlich sterilisiert werden, wie unten genauer dargestellt. Bevorzugt liegt in dem Übergangsbereich ein geringerer Druck an, als an dem Arbeitsbereich.

In dem Arbeitsraum kann beispielsweise ein Druck zwischen 0 und 10 bar anliegen und in dem Übergangsbereich beispielsweise ein Druck von ca. 5 bar.

Um den Übergangsbereich bzw. den Grauraum zu sterilisieren, ist es bevorzugt möglich, ein Sterilisationsmedium durch diesen strömen zu lassen.

Dazu ist es denkbar, dass ein Luftfederraum nicht nur mit einem Eingang, sondern auch mit einem Ausgang und insbesondere einem schaltbaren Ausgang versehen ist, sodass eine gleichmäßige Strömung erzeugt werden kann.

Die Luftfederauslässe können bevorzugt parallel geschaltet sein. Bevorzugt ist jedoch ein und insbesondere genau ein Gasdurchfluss vorgesehen.

Bei einer weiteren bevorzugten Ausführungsform weist die Ventileinrichtung und/oder die Ventilanordnung wenigstens eine Sensoreinrichtung auf. Derartige Sensoreinrichtungen in der Ventileinrichtung und/oder der Ventilanordnung können beispielsweise einen Druck in dem Arbeitsraum oder dem Steuerraum erfassen.

Daneben können Sensoreinrichtungen vorgesehen sein, welche eine Temperatur und/oder eine Konzentration eines Sterilisationsmediums erfassen, vorteilhaft um sicherzustellen und/oder zu protokollieren, dass eine ordnungsgemäße Sterilisation stattgefunden hat.

Bevorzugt sind mehrere der Ventileinrichtungen der erfindungsgemäßen Vorrichtung in der oben beschriebenen Weise ausgebildet.

Unter einer pneumatisch gesteuerten Ventileinrichtung wird insbesondere verstanden, dass Steuerluft oder dergleichen vorgesehen ist, um dieses Ventil von einer geschlossenen Stellung in eine geöffnete Stellung und/oder einer geöffneten Stellung in eine geschlossene Stellung zu schalten. In der geöffneten Stellung erfolgt bevorzugt eine Druckbeaufschlagung der Kunststoffvorformlinge mit einem Arbeitsdruck.

Bei einer bevorzugten Ausführungform weist die Ventileinrichtung einen beweglichen und insbesondere linearbeweglichen Ventilkolben auf, wobei in einer ersten Stellung dieses Ventilkolbens die Ventileinrichtung geschlossen ist und damit kein gasförmiges Medium durch die Ventileinrichtung zu der Beaufschlagungseinrichtung gelangt. Bevorzugt ist in einer zweiten Stellung dieses Ventilkolbens die Ventileinrichtung geöffnet und das gasförmige Medium bzw. die Blasluft gelangt zu der Beaufschlagungseinrichtung.

Bei dem fließfähigen Sterilisationsmedium kann es sich insbesondere um Wasserstoffperoxid oder Peressigsäure handeln. Im Stand der Technik existieren keine Ventile, die auf diese Weise vollständig sterilisierbar sind.

Bevorzugt weisen die Beaufschlagungseinrichtungen jeweils Blasdüsen auf oder sind Blasdüsen. Diese können dabei an eine Mündung der zu expandierenden Kunststoffvorformlinge oder auch am Bereich einer Blasform gelegt werden, um die Kunststoffvorformlinge so mit den einzelnen Drücken zu ihrer Expansion zu beaufschlagen.

Weiterhin weisen die Umformungsstationen bevorzugt jeweils Blasformen auf, innerhalb derer die Kunststoffvorformlinge zu den Kunststoffbehältnissen expandiert bzw. geblasen werden.

Besonders bevorzugt sind diese Blasformen dabei wenigstens zweiteilig und bevorzugt wenigstens dreiteilig aufgebaut und weisen beispielsweise zwei Seitenteile und ein Bodenteil auf.

Besonders bevorzugt ist das Druckluftreservoir welches den Luftdruck speichert mit den einzelnen Ventilanordnungen bzw. Ventilblöcken und genauer mit den einzelnen Ventileinrichtungen in Strömungsverbindung oder kann in eine solche gebracht werden. Dabei kann dies derart erfolgen, dass der entsprechende Arbeitsdruck stets an der Ventileinrichtung anliegt.

Bei einer bevorzugten Ausführungsform handelt es sich bei den Reservoirs um Ringkanäle. Besonders bevorzugt ist das wenigstens eine Reservoir und bevorzugt sind mehrere Reservoirs an dem drehbaren Träger, im Folgenden auch als Blasrad bezeichnet, angeordnet.

Bei einer weiteren vorteilhaften Ausführungsform erlauben alle Materialien der Ventileinrichtungen, welche mit dem Sterilisationsmedium in Kontakt kommen eine entsprechende Sterilisierung mit diesem Sterilisationsmedium.

Bei einer weiteren bevorzugten Ausführungsform weist die Vorrichtung eine Sterilisationseinrichtung auf, welche dazu geeignet ist, eine Ventileinrichtung oder mehrere Ventileinrichtungen mit dem Sterilisationsmedium zu beaufschlagen. Insbesondere können dabei diejenigen Strömungswege oder diejenigen Räume der Ventileinrichtung mit dem Sterilisationsmedium beaufschlagt werden, welche in einem Arbeitsbetrieb auch das Arbeitsfluid, insbesondere die Blasluft fördern.

Besonders bevorzugt ist der oben erwähnte Ventilkolben in einem aseptischen Raum der Vorrichtung angeordnet.

Bei einer bevorzugten Ausführungsform handelt es sich bei der Ventileinrichtung um ein aseptisches bzw. pneumatisch gesteuertes Ventil und wie unten genau beschrieben wird um ein balgloses Ventil.

Besonders bevorzugt ist sowohl ein Steuerraum dieser Ventileinrichtung, in welchem die Steuerluft zum Schalten des Ventils geführt wird, als auch ein Arbeitsraum dieser Ventileinrichtung, in welchem die Blasluft zum Expandieren der Kunststoffvorformlinge geführt und/oder wenigstens zeitweise gespeichert wird, vollständig sterilisierbar.

Bei aus dem Stand der Technik bekannten Ventilen sind die zur Verfügung stehenden Steuerkomponenten materialtechnisch in der Regel nicht in der Lage, sterilisiert zu werden, und es ist auch keine klar definierte Grenze eines Sterilraums definiert. Daher wird im Rahmen der Erfindung eine neuartige, insbesondere aseptische Ventileinrichtung vorgeschlagen.

Bei einer bevorzugten Ausführungsform weist die Ventileinrichtung ein Ventil, insbesondere ein Wegeventil und insbesondere ein aseptisches Wegeventil auf. Dieses Ventil steuert wiederum ein Kolbenventil und/oder den oben erwähnten Ventilkolben an (insbesondere um die Blasluft zu sperren oder sie zu der Beaufschlagungseinrichtung gelangen zu lassen).

Dabei ist es möglich, dass dieses Wegeventil mit einem Balg oder einer Membran versehen ist, sodass auf diese Weise eine aseptische Trennung zwischen einer Steuerluft und einem Reinraum stattfinden kann.

Bei einer bevorzugten Ausführungsform ist Ventileinrichtung balglos ausgeführt. Im Stand der Technik sind teilweise Faltenbälge vorgesehen, welche den aseptischen und den nicht-aseptischen Bereich der Ventileinrichtung voneinander trennen. Diese Bälge haben oft den Nachteil, dass sie von Sterilisationsmitteln angegriffen werden und auf diese Weise beschädigt werden. Außerdem halten derartiger Bälge oftmals Erwärmungen nicht stand. Dies bedeutet, dass in den Fällen, in denen man sehr schnell nach der Sterilisation des Ventils in einen Arbeitsbetrieb übergeht, das Material noch zu weich ist und es so zu Störungen oder Beschädigungen des Balgs führen kann.

Besonders bevorzugt sind der Steuerraum und der Arbeitsraum fluidtechnisch vollständig voneinander getrennt. Üblicherweise wird zum Steuern des Ventils nicht-aseptische Luft verwendet. Durch diese Ausgestaltung wird verhindert, dass diese Luft die eigentliche Arbeitsluft verunreinigen kann und auf diese Weise wird verhindert, dass letztlich verunreinigte Arbeitsluft in die Behältnisse gelangt.

Bei einer weiteren vorteilhaften Ausführungsform weist die Vorrichtung einen Reinraum auf, innerhalb dessen die umzuformenden Kunststoffvorformlinge transportiert werden. Insbesondere sind die Kunststoffvorformlinge während ihrer Expansion durch diesen Reinraum transportierbar. Bevorzugt sind auch die Umformungsstationen durch diesen Reinraum transportierbar.

Unter einem Reinraum wird ein Raumbereich bzw. ein Raum verstanden, innerhalb dessen die Luft einen höheren Reinheitsgrad bzw. eine geringere Anzahl an Keimen aufweist als in einer Umgebung. Dabei können unterschiedliche Reinheitsgrade vorgesehen sein, beispielsweise ein Reinheitsgrad, der gegenüber der Umgebung nur 1/10 oder auch nur 1/100 der Keimkonzentration aufweist. Bevorzugt ist dieser Reinraum gegenüber einer (unsterilen) Umgebung abgegrenzt.

Besonders bevorzugt werden die Kunststoffvorformlinge während ihrer Beaufschlagung mit dem gasförmigen Medium zu ihrer Expansion durch diesen Reinraum transportiert.

Bevorzugt weist der Reinraum wenigstens zwei diesen begrenzende Wandungen auf, die bevorzugt bezüglich einander beweglich sind.

Besonders bevorzugt weist die Vorrichtung eine Dichtungseinrichtung auf, welche den Reinraum gegenüber einer Umgebung abdichtet. Hierbei kann es sich beispielsweise um eine hydraulische Dichtung handeln. Besonders bevorzugt weist die Vorrichtung zwei Dichtungseinrichtungen, insbesondere zwei Wasserschlösser auf, welche dazu geeignet und bestimmt sind, den Reinraum gegenüber der Umgebung abzudichten.

Besonders bevorzugt befindet sich die oben erwähnte Kolbeneinrichtung der Ventileinrichtung, welche die Blasluft steuert stets innerhalb dieses Reinraums.

Besonders bevorzugt ist dabei die Vorrichtung derart ausgeführt, dass ein Druck der Arbeitsluft, welche zum Expandieren der Behältnisse verwendet wird, stets größer ist als ein Druck der Steuerluft, welche zum Betätigen der Ventileinrichtung dient.

Besonders bevorzugt ist ein Druck der Arbeitsluft größer als 5 bar, bevorzugt größer als 7 bar und bevorzugt größer als 10 bar. Besonders bevorzugt ist ein Druck der Arbeitsluft geringer als 60 bar, bevorzugt geringer als 50 bar, bevorzugt geringer als 40 bar und bevorzugt geringer als 30 bar.

Der Druck der Steuerluft ist besonders bevorzugt größer als 1 bar, bevorzugt größer als 2 bar, bevorzugt größer als 3 bar. Weiterhin ist der Druck der Steuerluft geringer als 15 bar, bevorzugt geringer als 12 bar und besonders bevorzugt geringer als 10 bar. Durch die Ausgestaltung dieser Druckverhältnisse wird erreicht, dass auch bei einer Beschädigung der Membran oder des Balgs kein Eintritt der nicht-aseptischen Steuerluft zu der aseptischen Arbeitsluft auftritt.

Bei einer weiteren vorteilhaften Ausführungsform weist die Vorrichtung ein zweites Druckluftreservoir auf, welches dazu geeignet und bestimmt ist, Druckluft unter einem zweiten vorgebbaren Druck bereitzustellen, wobei dieser zweite Druck höher ist als der erste Druck. So kann es sich bei dem zweiten Druck beispielsweise um einen Fertigblasdruck handeln, mit dem das Behältnis bevorzugt endgültig ausgeformt und dessen endgültige Gestalt festgelegt wird.

Bei einer weiteren vorteilhaften Ausführungsform weist die Vorrichtung ein drittes Druckluftreservoir auf, welches dazu geeignet und bestimmt ist, Druckluft unter einem dritten vorgegebenen Druck bereitzustellen, wobei dieser dritte Druck bevorzugt größer ist als der erste Druck und bevorzugt geringer als der zweite Druck.

Bei diesem Druck kann es sich insbesondere um den sogenannten Zwischenblasdruck handeln, der bei neueren Verfahren zum Herstellen von Kunststoffbehältnissen verwendet wird. Besonders bevorzugt ist auch das zweite Druckluftreservoir und das dritte Druckluftreservoir über Ventileinrichtungen in Strömungsverbindung mit der Beaufschlagungseinrichtung und letztlich dem zu expandierenden Kunststoffvorformling bringbar.

Besonders bevorzugt handelt es sich auch bei den Ventileinrichtungen, die die Beaufschlagung des Behältnisses mit dem zweiten Druck und dem dritten Druck steuern, um oben beschriebene Ventile, die besonders bevorzugt vollständig sterilisierbar sind.

Besonders bevorzugt handelt es sich auch bei dem zweiten Druckluftreservoir und/oder dem dritten Druckluftreservoirs um einen Ringkanal, der an dem Blasrad angeordnet ist.

Bei der weiteren vorteilhaften Ausführungsform weisen die Umformungsstationen jeweils stangenartige Körper, insbesondere sogenannte Reckstangen auf, welche in die Kunststoffvorformlinge einführbar sind, um diese in deren Längsrichtung zu dehnen.

Bei einer weiteren bevorzugten Ausführungsform ist der Kolben der Ventileinrichtung (bzw. der Ventilkolben) teilweise oder vollständig druckkompensiert. Dies bedeutet, dass keine oder nur eine sehr kleine resultierende Kraft auf den Kolben aufgrund der anliegenden Drücke wirkt.

Bei einer weiteren vorteilhaften Ausführungsform ist eine Führungseinrichtung des (Ventil)Kolbens vorgesehen. Diese ist bevorzugt derart angeordnet, dass ein Sterilisationsmedium den Kolben überall umschließend erreichen kann. Hiervon ausgenommen kann eine entsprechende Dichtung sein, da diese Stelle dann durch das Bewegen während des Sterilisierens ebenfalls sterilisiert werden kann.

Besonders bevorzugt weist die Ventileinrichtung wenigstens eine Sensoreinrichtung auf. So kann es sich beispielsweise bei der Sensoreinrichtung um eine Druckmesseinrichtung handeln, welche einen Druck der Arbeitsluft und/oder der Steuerluft innerhalb der Ventileinrichtung bestimmt. Auf diese Weise kann sichergestellt werden, dass der Druck der Arbeitsluft stets höher ist als der Druck der Steuerluft.

Bei einer weiteren bevorzugten Ausführungsform können Sensoreinrichtungen an oder in der Ventileinrichtung vorgesehen sein, welche beispielsweise einen Druck oder eine Temperatur und/oder eine Konzentration des Sterilisationsmittels ermitteln. Auf diese Weise ist es möglich, die Sterilisation der Ventileinrichtung zu protokollieren und/oder zu überprüfen, ob eine ordnungsgemäße Sterilisation stattgefunden hat.

Die vorliegende Erfindung ist weiterhin auf ein Verfahren zum Umformen von Kunststoffvorformlingen zu Kunststoffbehältnissen gerichtet, wobei eine Transporteinrichtung die Kunststoffvorformlinge entlang eines vorgegebenen Transportpfads transportiert und wobei die Transporteinrichtung einen beweglichen und insbesondere einen drehbaren Transportträger aufweist, an dem eine Vielzahl von Umformungsstationen zum Umformen der Kunststoffvorformlinge angeordnet ist.

Dabei weisen diese Umformungsstationen jeweils Beaufschlagungseinrichtungen auf, welche die Kunststoffvorformlinge zu ihrer Expansion mit einem fließfähigen und insbesondere gasförmigen Medium beaufschlagen. Weiterhin weist die Vorrichtung wenigstens ein erstes Druckluftreservoir auf, welches Druckluft unter einem ersten vorgegebenen Druck speichert und jede Umformungsstation weist eine Ventilanordnung mit einer Vielzahl von Ventileinrichtungen auf, um die Kunststoffvorformlinge mit wenigstens zwei unterschiedlichen Drücken zu beaufschlagen.

Weiterhin wird eine dieser Ventileinrichtungen pneumatisch gesteuert und/oder betätigt.

Erfindungsgemäß weist diese Ventileinrichtung einen Arbeitsraum auf, welcher das zur Beaufschlagung der Kunststoffvorformlinge verwendete gasförmigen Mediums aufnimmt und/oder bereitstellt sowie einen zwischen wenigstens zwei Position bewegbaren Ventilkolben (insbesondere um die Ventileinrichtung zwischen einer geschlossenen und einer geöffneten Stellung zu schalten) wobei die Ventileinrichtung weiterhin einen Steuerraum aufweist, der von einem gasförmigen Steuermedium zur Bewegung des Ventilkolbens beaufschlagt wird und wobei zwischen dem Arbeitsraum und dem Steuerraum ein Übergangsbereich angeordnet ist.

Bei einem weiteren bevorzugten Verfahren herrscht in dem Arbeitsraum der Ventileinrichtung, der zur Aufnahme des die Kunststoffvorformlinge beaufschlagenden gasförmigen Mediums dient, während eines Arbeitsbetriebs ein höherer Druck als in dem Steuerraum, der zu Aufnahme eines Steuerfluids dient, welches einen Ventilkolben der Ventileinrichtung bewegt.

Bevorzugt wird die Vorrichtung zeitweise in einem Arbeitsbetrieb betrieben, in dem Kunststoffvorformlinge zu Kunststoffbehältnissen umgeformt werden und zeitweise in einem Sterilisationsbetrieb, in welchem Komponenten der Vorrichtungen und insbesondere die Ventileinrichtungen sterilisiert werden.

Die vorliegende Erfindung ist weiterhin auf die Verwendung einer pneumatisch gesteuerten Ventileinrichtung zum Betrieb einer Umformungseinrichtung zum Umformen von Kunststoffvorformlingen zu Kunststoffbehältnissen und insbesondere einer Streckblasmaschine gerichtet.

Erfindungsgemäß weist diese Ventileinrichtung einen Arbeitsraum zur Aufnahme des zur Beaufschlagung der Kunststoffvorformlinge verwendeten gasförmigen Mediums auf sowie einen zwischen wenigstens zwei Position bewegbaren Ventilkolben und die Ventileinrichtung weist weiterhin einen Steuerraum auf, der von einem gasförmigen Steuermedium zur Bewegung des Ventilkolbens beaufschlagbar ist und zwischen dem Arbeitsraum und dem Steuerraum ist ein Übergangsbereich angeordnet.

Weitere Vorteile und Ausführungsformen ergeben sich aus den beigefügten Zeichnungen:
Darin zeigen:
- Fig. 1: eine schematische Darstellung einer erfindungsgemäßen Vorrichtung;
- Fig. 2: eine schematische Darstellung einer Ventileinrichtung in einem geschlossenen Zustand; und
- Fig. 3: eine Darstellung der Ventileinrichtung in einem geöffneten Zustand.

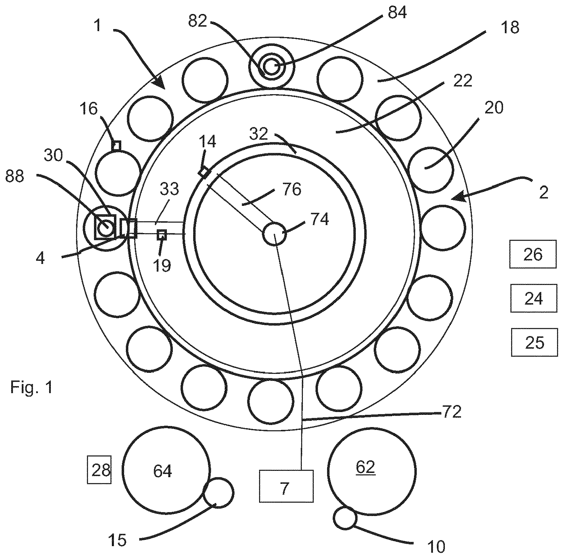

Fig. 1 zeigt eine Vorrichtung 1 zum Umformen von Kunststoffvorformlingen 10 zu Kunststoffbehältnissen 15. Diese Vorrichtung weist eine Transporteinrichtung 2 zum Transportieren der Kunststoffvorformlinge mit einen drehbaren Träger 22 auf, an dem eine Vielzahl von Umformungsstationen 20 angeordnet ist. Diese einzelnen Umformungsstationen weisen jeweils Blasformeinrichtungen 82 auf, die in ihrem Inneren einen Hohlraum zum Expandieren der Kunststoffvorformlinge ausbilden.

Das Bezugszeichen 84 kennzeichnet eine Beaufschlagungseinrichtung, welche zum Expandieren der Kunststoffvorformlinge 10 dient. Hierbei kann es sich beispielsweise um eine Blasdüse handeln, welche an eine Mündung der Kunststoffvorformlinge 10 anlegbar ist, um diese so zu expandieren. Daneben wäre es auch denkbar, dass die Blasdüse an der Blasformeinrichtung abdichtet. Bevorzugt ist diese Beaufschlagungseinrichtung in einer Längsrichtung und bevorzugt ausschließlich in einer Längsrichtung der Kunststoffvorformlinge bewegbar.

Das Bezugszeichen 4 kennzeichnet eine Ventilanordnung wie einen Ventilblock, der bevorzugt eine Vielzahl von Ventileinrichtungen aufweist, welche die Beaufschlagung der Kunststoffvorformlinge mit unterschiedlichen Druckniveaus steuern. Bevorzugt weist jede Umformungsstation einen derartigen Ventilblock auf.

Bevorzugt ist auch an jeder dieser Ventilanordnungen eine Drosseleinrichtung angeordnet, welche den Strömungsquerschnitt der den Kunststoffvorformlingen unter wenigstens einem Druck zugeführten Druckluft steuert. Wie oben erwähnt sind wenigstens einige dieser Ventileinrichtungen und bevorzugt alle dieser Ventileinrichtungen sterilisierbar.

Bei einem bevorzugten Verfahren werden die Kunststoffvorformlinge zunächst mit einem Vorblasdruck P1 beaufschlagt, anschließend mit mindestens einem Zwischenblasdruck Pi, der höher ist als der Vorblasdruck und schließlich mit einem Fertigblasdruck P2, der höher ist als der Zwischenblasdruck Pi1. Besonders bevorzugt werden die Kunststoffvorformlinge mit einem weiteren Zwischenblasdruck Pi2 beaufschlagt, der größer ist als der Druck Pi1 jedoch kleiner als der Druck P2.

Nach der Expansion der Kunststoffbehältnisse werden bevorzugt die Drücke bzw. die Druckluft wieder von dem Behältnis in die einzelnen Druckreservoirs zurückgeführt.

Bevorzugt weist die Vorrichtung daher eine Luftrecyceleinrichtung auf, welche dazu geeignet und bestimmt ist, Druckluft von den einzelnen Umformungsstationen in Druckluftreservoirs, insbesondere solche Druckluftreservoirs, welche ein geringeres Druckniveau aufnehmen, zurückzuführen.

Das Bezugszeichen 88 kennzeichnet eine Reckstange, die dazu dient, die Kunststoffvorform-linge in ihrer Längsrichtung zu dehnen. Bevorzugt weisen sämtliche Umformungsstationen derartige Blasformen 82 sowie Reckstangen 88 auf. Diese Reckstange ist bevorzugt Bestandteil einer mit 30 bezeichneten Reckeinrichtung. Die Reckstange ist (bevorzugt ebenfalls ausschließlich) in der Längsrichtung der Kunststoffvorformlinge 10 beweglich.

Bevorzugt liegt die Anzahl dieser Umformungsstationen 4 zwischen 2 und 100, bevorzugt zwischen 4 und 60, bevorzugt zwischen 6 und 40.

Die Kunststoffvorformlinge 10 werden über eine erste Transporteinrichtung 62 wie insbesondere aber nicht ausschließlich einen Transportstern der Vorrichtung zugeführt. Über eine zweite Transporteinrichtung 64 werden die Kunststoffbehältnisse 15 abtransportiert.

Das Bezugszeichen 7 kennzeichnet eine Druckbereitstellungseinrichtung wie etwa einen Kompressor oder auch einen Druckluftanschluss. Die Druckluft wird über eine Verbindungsleitung 72 zu einem Drehverteiler 74 gefördert und von diesem über eine weitere Leitung 76 an das erste Druckluftreservoir 32 angegeben, bei dem es sich hier um einen Ringkanal handelt.

Damit dient bevorzugt dieser Drehverteiler zu dem Zweck, Luft von einem stationären Teil der Vorrichtung in einen sich drehenden Teil der Vorrichtung zu führen.

Neben diesem dargestellten Ringkanal 32 sind bevorzugt noch weitere Ringkanäle vorgesehen, die in der in Fig. 1 gezeigten Darstellung jedoch durch den Ringkanal 32 verdeckt sind, beispielsweise darunterliegen. So ist bevorzugt je ein Druckreservoir zur Speicherung des Drucks P2 sowie der Zwischenblasdrücke Pi1 und Pi2 und des Drucks P1 vorhanden.

Das Bezugszeichen 33 kennzeichnet eine Verbindungsleitung, welche die Druckluft an eine Umformungsstation 20 bzw. deren Ventilblock 4 abgibt. Bevorzugt ist jeder der Ringkanäle über entsprechende Verbindungsleitungen mit sämtlichen Umformungsstationen verbunden. Diese Verbindungsleitung 33 ist bevorzugt in dem drehenden Teil der Vorrichtung angeordnet.

Das Bezugszeichen 18 kennzeichnet schematisch einen optionalen Reinraum, der hier bevorzugt ringförmig ausgebildet ist und den Transportpfad der Kunststoffvorformlinge 10 umgibt. Bevorzugt ist eine (geometrische) Drehachse bezüglich derer der Transportträger 22 drehbar ist, außerhalb des Reinraums 18 angeordnet. Bevorzugt ist der Reinraum 18 mit einer Dichtungseinrichtung, welche bevorzugt wenigstens zwei Wasserschlösser aufweist, gegen-über der unsterilen Umgebung abgedichtet. Bevorzugt ist dieser Reinraum ring-oder torusförmig ausgebildet

Weiterhin weist die Vorrichtung bevorzugt eine (in Fig. 1 nicht gezeigte) Deckeleinrichtung auf, welche den Reinraum 18 nach oben hin begrenzt. Diese Deckeleinrichtung ist dabei bevorzugt an wenigstens einer der Reckeinrichtungen 30 angeordnet.

Die Vorrichtung weist eine Vielzahl von Mess- und/oder Sensoreinrichtungen auf, welche zur Steuerung der Vorrichtung dienen. Das Bezugszeichen 14 kennzeichnet eine Druckmesseinrichtung, welche einen Luftdruck innerhalb des Druckluftreservoirs 32 misst. Bevorzugt weisen auch die übrigen Druckluftreservoirs entsprechende Druckmesseinrichtungen auf.

Das Bezugszeichen 16 kennzeichnet eine weitere Druckmesseinrichtung, welche einen Luftdruck insbesondere einen Behältnisinnendruck des zu expandierenden Kunststoffvorformlings misst. Bevorzugt ist jeder Umformungsstation eine solche Druckmesseinrichtung zugeordnet.

Bevorzugt weisen auch die Ventileinrichtungen selbst jeweils wenigstens eine und bevorzugt mehrere Sensoreinrichtungen wie Druckmesseinrichtungen auf. Daneben können die Ventileinrichtungen auch Sensoreinrichtungen zur Erfassung der Konzentration eines Sterilisationsmedium aufweisen.

Bevorzugt weist die Vorrichtung eine Sterilisationseinrichtung auf, welche dazu geeignet und bestimmt ist, die einzelnen Ventileinrichtungen mit einem fließfähigen Sterilisationsmedium zu beaufschlagen. Bevorzugt findet dabei diese Sterilisation in einem von einem Arbeitsbetrieb der Vorrichtung unterschiedlichen Sterilisationsbetrieb statt.

Das Bezugszeichen 19 kennzeichnet ebenfalls schematisch eine Durchflussmesseinrichtung, welche einen Durchfluss der Blasluft von einem Druckluftreservoir zu dem Ventilblock 4 einer Umformungsstation 20 bestimmt. Bevorzugt sind entsprechende Durchflussmesseinrichtungen jeweils zwischen einem Druckluftreservoir und allen Umformungsstationen angeordnet.

Auch zwischen den weiteren Druckluftreservoirs und den jeweiligen Umformungsstationen können weitere Durchflussmeseinrichtungen zugeordnet sein.

Weiterhin sind bevorzugt auch Positionserfassungseinrichtungen vorgesehen, welche Positionen der Reckstangen der einzelnen Umformungsstationen erfassen können.

Das Bezugszeichen 24 kennzeichnet eine Steuerungseinrichtung welche die Vorrichtung 1 steuert und insbesondere regelt. Diese Steuerungseinrichtung ist dabei bevorzugt auch in der Lage, Arbeitsparameter der Vorrichtung und insbesondere Einstellungen der Drosseleinrichtung zu ändern.

So steuert die Steuerungseinrichtung insbesondere die einzelnen Ventileinrichtungen und da-mit die Beaufschlagung der Kunststoffvorformlinge mit den einzelnen Druckniveaus. Daneben steuert die Steuerungseinrichtung bevorzugt auch eine Bewegung der Reckstangen der einzelnen Umformungsstationen. Bevorzugt steuert die Steuerungseinrichtung auch Bewegungen der Beaufschlagungseinrichtungen d.h. der Blasdüsen. Wie erwähnt kann diese Steuerungseinrichtung bevorzugt auch die Einstellungen der einzelnen Drosseleinrichtungen ein-stellen.

Die Steuerungseinrichtung ist bevorzugt dazu geeignet, die Zeitpunkte, zu denen die Beaufschlagungseinrichtungen an die Kunststoffvorformlinge angelegt werden und/oder die Zeitpunkte, zu denen die Blasformeinrichtungen wieder von den Kunststoffvorformlingen abgehoben werden zu steuern und insbesondere auch, diese Zeitpunkte zu verändern.

Das Bezugszeichen 26 kennzeichnet eine Speichereinrichtung, in der insbesondere Messgrößen erfasst werden, insbesondere Druckwerte und Durchflusswerte, aber auch entsprechen-de Arbeitsparameter. Bevorzugt werden diese jeweiligen Werte mit einer zeitlichen Zuordnung gespeichert.

Bevorzugt können diese Werte kontinuierlich und insbesondere über lange Zeiträume eines Maschinenbetriebs gespeichert. Die Steuerungseinrichtung steuert bzw. regelt auch unter Berücksichtigung dieser aufgenommenen Messwerte die Vorrichtung.

Das Bezugszeichen 28 kennzeichnet grob schematisch eine Inspektionseinrichtung zum Inspizieren der gefertigten Behältnisse.

Das Bezugszeichen 25 kennzeichnet eine Anzeigeeinrichtung, welche zur Ausgabe von Informationen an einen Maschinenbediener dient. Mittels dieser Anzeigeeinrichtung können etwa gemessene Druck(verlaufs)kurven ausgegeben werden.

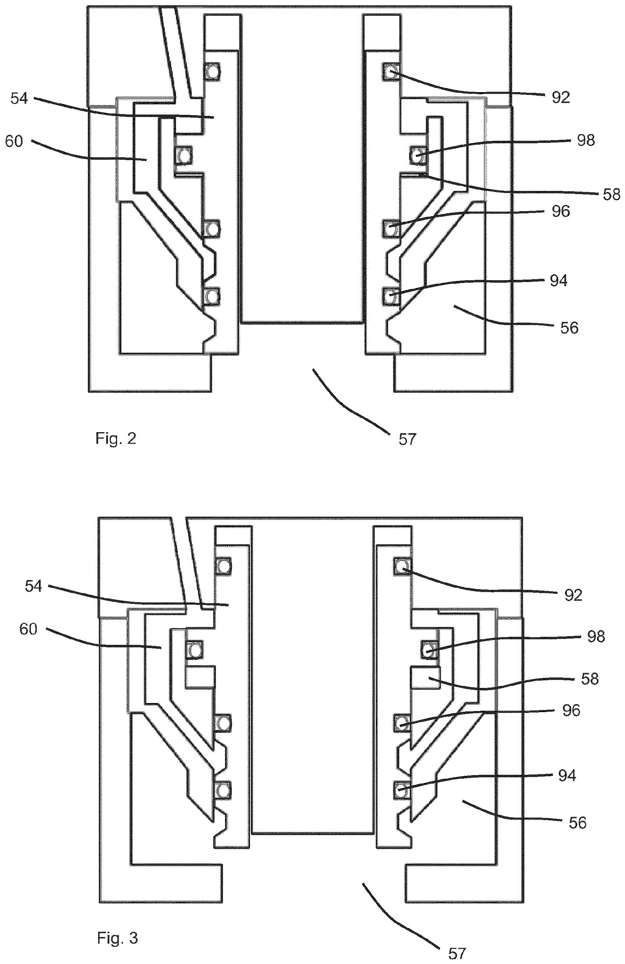

Die Fig. 2 und 3 zeigen eine erfindungsgemäße Ventileinrichtung 50, einmal in einem geschlossenen Zustand (Fig. 2) und einmal in einem geöffneten Zustand (Fig. 3). Dabei bezieht sich das Bezugszeichen 56 auf einen Arbeitsraum, der mit dem gasförmigen Medium, welches zur Expansion der Kunststoffvorformlinge dient, beaufschlagt werden kann. Bevorzugt liegt dabei dieser Blasdruck permanent an diesem Arbeitsraum 56 an. Das Bezugszeichen 57 kennzeichnet eine Ausgangsöffnung der Ventileinrichtung, welche mit der Beaufschlagungseinrichtung und damit letztlich dem Kunststoffvorformling in Strömungsverbindung steht.

Das Bezugszeichen 54 kennzeichnet ein Ventilkolben, der in Fig. 2 in einer geschlossenen Stellung und in Fig. 3 in einer geöffneten Stellung der Ventileinrichtung 50 gezeigt ist.

Das Bezugszeichen 58 kennzeichnet einen Steuerraum, der mit (unsteriler) Steuerluft beaufschlagt werden kann, um so den Ventilkolben 54 zu bewegen.

Durch den Aufbau der Ventileinrichtung kann diese in drei Zonen unterteilt werden, nämlich eine erste Zone, in der sich der Arbeitsraum 56 befindet, einen Übergangsbereich bzw. eine zweite Zone oder Übergangszone und eine dritte Zone, in der sich der Steuerraum befindet.

Das Bezugszeichen 94 kennzeichnet eine Dichtungseinrichtung und insbesondere ein O-Ring, welcher den Arbeitsraum gegenüber dem Zwischenbereich 60 abdichtet. Das Bezugszeichen 96 kennzeichnet eine Dichtungseinrichtung und bevorzugt ebenfalls einen O-Ring oder dergleichen, welche den Steuerraum 58 gegenüber dem Übergangsbereich 60 abdichtet.

In dem Übergangsbereich wird ein umlaufendes Volumen 60 ausgebildet, welches bevorzugt von steriler Luft beaufschlagt wird. Die Bezugszeichen 92 und 98 kennzeichnen weitere Dichtungseinrichtungen, welche zusätzlich diesen Übergangsbereich 60 sowohl gegenüber dem Steuerraum 58 abdichten.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

## Patentansprüche

1. Vorrichtung zum Umformen von Kunststoffvorformlingen (10) zu Kunststoffbehältnissen, mit einer Transporteinrichtung (2), welche die Kunststoffvorformlinge (10) entlang eines vorgegebenen Transportpfads transportiert, wobei die Transporteinrichtung (2) einen Transportträger (22) aufweist, an dem eine Vielzahl von Umformungsstationen (20) zum Umformen der Kunststoffvorformlinge (10) aufweist, wobei diese Umformungsstationen jeweils Beaufschlagungseinrichtungen (84) aufweisen, welche die Kunststoffvorformlinge zu ihrer Expansion mit einem fließfähigen und insbesondere gasförmigen Medium beaufschlagen und wobei die Vorrichtung (1) wenigstens ein erstes Druckluftreservoir (32) aufweist, welches dazu geeignet und bestimmt ist, Druckluft unter einem ersten vorgegebenen Druck bereitzustellen und jede Umformungsstation (20) eine Ventilanordnung (4) mit einer Vielzahl von Ventileinrichtungen (50) aufweist, um die Kunststoffvorformlinge mit wenigstens zwei Drücken zu beaufschlagen, wobei wenigstens eine dieser Ventileinrichtungen (50) eine pneumatisch gesteuerte Ventileinrichtung (50) ist,
**dadurch gekennzeichnet, dass**
diese Ventileinrichtung (50) einen Arbeitsraum (56) zur Aufnahme des zur Beaufschlagung der Kunststoffvorformlinge (10) verwendeten gasförmigen Mediums aufweist sowie einen zwischen wenigstens zwei Position bewegbaren Ventilkolben (54) aufweist und die Ventileinrichtung (50) weiterhin einen Steuerraum (58) aufweist, der von einem gasförmigen Steuermedium zur Bewegung des Ventilkolbens (54) beaufschlagbar ist und wobei zwischen dem Arbeitsraum (56) und dem Steuerraum (58) mindestens ein Übergangsbereich (60) angeordnet ist.

2. Vorrichtung (1) nach Anspruch 1,
**dadurch gekennzeichnet, dass**
der Arbeitsraum (56) und/oder der Übergangsbereich (60) und bevorzugt der Arbeitsraum und der Übergangsraum (60) sterilisierbar sind.

3. Vorrichtung (1) nach wenigstens einem der vorangegangenen Ansprüche,
**dadurch gekennzeichnet, dass**
der Steuerraum (58) gegenüber dem Übergangsbereich (60) mittels wenigstens einer ersten Dichtungseinrichtung (96,98) abgedichtet ist und/oder der Übergangsbereich (60) gegenüber dem Arbeitsraum mit wenigstens einer zweiten Dichtungseinrichtung (94) abgedichtet ist.

4. Vorrichtung nach dem vorangegangenen Anspruch,
**dadurch gekennzeichnet, dass**
die erste Dichtungseinrichtung (92, 94), welche den Steuerraum gegenüber dem Übergangsbereich (60) abdichtet, derart angeordnet ist, dass sie niemals einen aseptischen Bereich des Arbeitsraums kontaktiert.

5. Vorrichtung (1) nach wenigstens einem der vorangegangenen Ansprüche,
**dadurch gekennzeichnet, dass**
der Übergangsbereich (60) als Druckfeder zum Vorspannen der Kolbeneinrichtung (54) wirkt.

6. Vorrichtung (1) nach wenigstens einem der vorangegangenen Ansprüche,
**dadurch gekennzeichnet, dass**
die Ventilanordnung und/oder die Ventileinrichtung wenigstens eine Sensoreinrichtung aufweist.

7. Vorrichtung (1) nach Anspruch 1,
**dadurch gekennzeichnet, dass**
die Ventileinrichtung balglos ausgeführt ist.

8. Vorrichtung (1) nach wenigstens einem der vorangegangenen Ansprüche,
**dadurch gekennzeichnet, dass**
die Vorrichtung (1) einen Reinraum aufweist, innerhalb dessen die umzuformenden Kunststoffvorformlinge transportiert werden.

9. Vorrichtung nach wenigstens einem der vorangegangenen Ansprüche,
**dadurch gekennzeichnet, dass**
ein Steuerdruck zum Ansteuern der Ventileinrichtung niedriger ist als ein Druck des gasförmigen Mediums zum Expandieren der Kunststoffvorformlinge.

10. Vorrichtung nach wenigstens einem der vorangegangenen Ansprüche,
**dadurch gekennzeichnet, dass**
die Vorrichtung ein zweites Druckluftreservoir aufweist, welches dazu geeignet und bestimmt ist, Druckluft unter einem zweiten vorgegebenen Druck (pi) bereitzustellen, wobei dieser zweite Druck höher ist als der erste Druck (p1).

11. Vorrichtung (1) nach wenigstens einem der vorangegangenen Ansprüche,
**dadurch gekennzeichnet, dass**
die Vorrichtung (1) sowohl in einem Arbeitsbetrieb betreibbar ist, in welchem die Kunststoffvorformlinge (10) umgeformt werden als auch in einem Sterilisationsbetrieb, in welchem die Vorrichtung (1) und/oder die Ventileinrichtung sterilisiert wird wodurch sich bevorzugt die Betriebe unterscheiden.

12. Verfahren zum Umformen von Kunststoffvorformlingen (10) zu Kunststoffbehältnissen, wobei eine Transporteinrichtung (2) die Kunststoffvorformlinge (10) entlang eines vorgegebenen Transportpfads (P) transportiert, wobei die Transporteinrichtung (2) einen Transportträger (22) aufweist, an dem eine Vielzahl von Umformungsstationen (20) zum Umformen der Kunststoffvorformlinge (10) angeordnet ist, wobei diese Umformungsstationen jeweils Beaufschlagungseinrichtungen (84) aufweisen, welche die Kunststoffvorformlinge (10) zu ihrer Expansion mit einem fließfähigen und insbesondere gasförmigen Medium beaufschlagen und wobei die Vorrichtung (1) wenigstens ein erstes Druckluftreservoir (32) aufweist, welches Druckluft unter einem ersten vorgegebenen Druck bereitstellt und jede Umformungsstation (20) eine Ventilanordnung (4) mit einer Vielzahl von Ventileinrichtungen (52, 54) aufweist, um die Kunststoffvorformlinge mit den wenigstens zwei Drücken zu beaufschlagen wobei wenigstens eine dieser Ventileinrichtungen (50) pneumatisch gesteuert wird,
**dadurch gekennzeichnet, dass**
diese Ventileinrichtung (50) einen Arbeitsraum (56) aufweist, welcher das zur Beaufschlagung der Kunststoffvorformlinge (10) verwendete gasförmigen Medium aufnimmt sowie einen zwischen wenigstens zwei Position bewegbaren Ventilkolben (54) und die Ventileinrichtung (50) weiterhin einen Steuerraum (58) aufweist, der von einem gasförmigen Steuermedium zur Bewegung des Ventilkolbens (54) beaufschlagt wird und wobei zwischen dem Arbeitsraum (56) und dem Steuerraum (58) ein Übergangsbereich (60) angeordnet ist.

13. Verwendung einer pneumatisch gesteuerten Ventileinrichtung (50) zum Betrieb einer Umformungseinrichtung zum Umformen von Kunststoffvorformlingen zu Kunststoffbehältnissen,
**dadurch gekennzeichnet, dass**
diese Ventileinrichtung (50) einen Arbeitsraum (56) zur Aufnahme des zur Beaufschlagung der Kunststoffvorformlinge (10) verwendeten gasförmigen Mediums aufweist sowie einen zwischen wenigstens zwei Position bewegbaren Ventilkolben (54) und die Ventileinrichtung (50) weiterhin einen Steuerraum (58) aufweist, der von einem gasförmigen Steuermedium zur Bewegung des Ventilkolbens (54) beaufschlagbar ist und wobei zwischen dem Arbeitsraum (56) und dem Steuerraum (58) ein Übergangsbereich (60) angeordnet ist.
